# EUROPEAN PATENT SPECIFICATION

(11) **EP 3 154 599 B1**
(45) Date of publication and mention of the grant of the patent: **14.08.2019**
(21) Application number: 15729885.2
(22) Date of filing: 09.06.2015
(51) Int. Cl.: A61L 27/18, A61L 27/22, A61L 27/38

(54) **HYBRID COMPOSITION**
HYBRIDE ZUSAMMENSETZUNG
COMPOSITION HYBRIDE

(30) Priority: 12.06.2014 GB 201410506
(43) Date of publication of application: 19.04.2017
(73) Proprietor: Cell Therapy Limited, Swansea SA2 8PP (GB)
(72) Inventor: EVANS, Martin John, Swansea West Glamorgan SA2 8PP (GB); REGINALD, Ajan, Swansea West Glamorgan SA2 8PP (GB); SULTAN, Sabena, Swansea West Glamorgan SA2 8PP (GB)
(74) Representative: J A Kemp
(86) International application number: PCT/GB2015/051672
(87) International publication number: WO 2015/189586

(56) References cited:
- WO-A2-2008/142129
- US-A1- 2013 004 541

## Description

### Field of the Invention

The invention relates to a novel chemical composition comprising living cells combined with biocompatible fibres and biocompatible components which are optimally chemically formulated to repair damaged human or animal tissue.

### Background to the Invention

A variety of different cell types are used in repairing damaged tissue. Once administered to the patient, the cells typically exert their therapeutic effects through paracrine signalling and by promoting survival, repair and regeneration of the neighbouring cells in the damaged tissue. Hybrid compositions for tissue repair, comprising cells and fibres, are disclosed in US2013/004541 A1 and WO2008/142129 A2.

The repair of damaged cells by therapeutic cells is often inefficient because (1) the cells can only access and repair the surface of the damaged tissue, but cannot reach deeper parts of the damaged tissue, (2) the cells remain in the optimal location for repair for a limited time period and (3) the local microenvironment in which the cells are placed is not optimal to promote repair and regeneration. These limitations often result in a sub-optimal or partial repair of the damaged tissue.

### Summary of the Invention

The inventors have surprisingly found that a unique hybrid composition of living cells, biocompatible fibres and biocompatible components is more effective at repairing damaged tissues. For instance, the biocompatible fibres and components are capable of providing the cells with access to the optimal location to effect optimal repair of damaged tissue. The cells may, for example, reach deeper parts of the damaged tissue. The biocompatible fibres and components are also capable of retaining the cells at the optimal location to effect optimal repair of damaged tissue. The composition also creates its own optimal microenvironment for optimal repair and regeneration.

The invention therefore provides a hybrid composition, as defined by claim 1.

The invention also provides:
- a method of producing a hybrid composition of the invention as defined by claim 10; or
- a hybrid composition of the invention for use in a method of repairing a damaged tissue in a patient, as defined by claim 11.

### Brief Description of the Figures

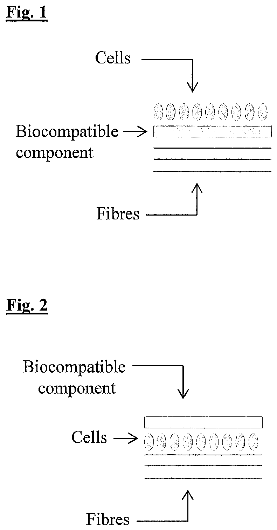

Figure 1 shows the general structure of a hybrid composition of the invention in which the therapeutic cells are attached to the one or more fibres using a biocompatible component.
Figure 2 shows the general structure of a hybrid composition of the invention in which the therapeutic cells are embedded in the biocompatible component.

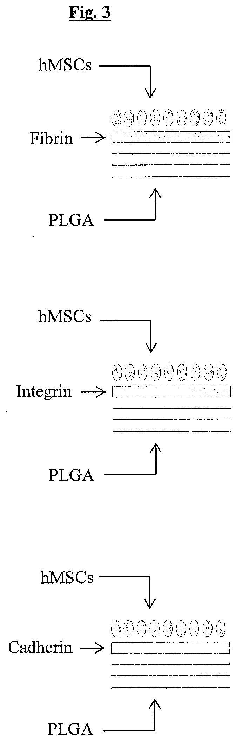

Figure 3 shows the preferred structures of hybrid compositions of the type shown in Figure 1. In each case, the therapeutic cells are human mesenchymal stem cells (MSCs) and the one or more fibres comprise poly(D,L-lactide-co-glycolide) (PLGA). The biocompatible component comprises fibrin, integrin or adherin.

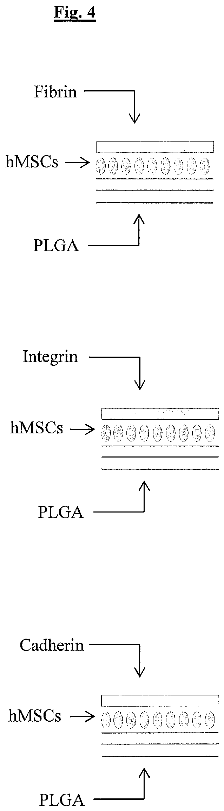

Figure 4 shows the preferred structures of hybrid compositions of the type shown in Figure 2. In each case, the therapeutic cells are human mesenchymal stem cells (MSCs) and the one or more fibres comprise poly(D,L-lactide-co-glycolide) (PLGA). The biocompatible component comprises fibrin, integrin or cadherin.

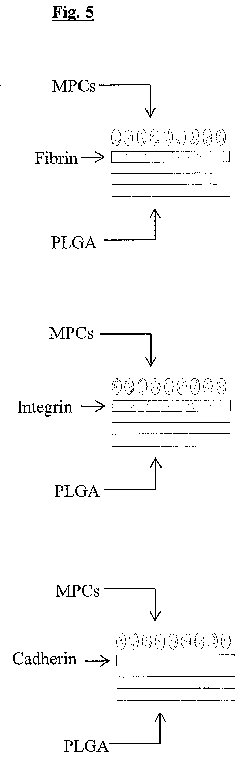

Figure 5 shows the preferred structures of hybrid compositions of the type shown in Figure 1. In each case, the therapeutic cells are mesenchymal precursor cells (MPCs) and the one or more fibres comprise poly(D,L-lactide-co-glycolide) (PLGA). The biocompatible component comprises fibrin, integrin or cadherin.

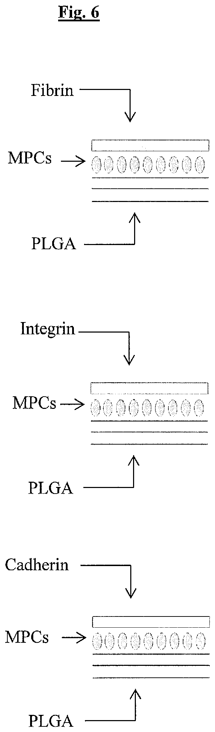

Figure 6 shows the preferred structures of hybrid compositions of the type shown in Figure 2. In each case, the therapeutic cells are mesenchymal precursor cells (MPCs) and the one or more fibres comprise poly(D,L-lactide-co-glycolide) (PLGA). The biocompatible component comprises fibrin, integrin or cadherin.

### Detailed Description of the Invention

It is to be understood that different applications of the disclosed products and methods may be tailored to the specific needs in the art.

### Composition of the invention

The invention provides a hybrid composition. The composition is a hybrid because it comprises living cells and non-living fibres and components.

The composition is suitable for repairing damaged tissue. The composition may be implanted or injected into damaged tissue. The composition may be adhered to or anchored to damaged tissue. This is discussed in more detail below with reference to the therapeutic embodiments of the invention.

### Biocompatible fibres

The composition of the invention comprises one or more biocompatible fibres. A fibre is biocompatible if it does not cause any adverse reactions or side effects when contacted with a damaged tissue.

Any number of biocompatible fibres may be present in the composition. The composition may comprise only one fibre. The composition typically comprises more than one fibre, such at least 2, at least 5, at least 10, at least 20, at least 30, at least 40, at least 50, at least 100, at least 200, at least 500 fibres, at least 1000 fibres or even more fibres.

Suitable biocompatible fibres are known in the art. The one or more biocompatible fibres may be natural or synthetic. Preferred biocompatible fibres include, but are not limited to, cellulose fibres, collagen fibres, collagen-glycosaminoglycan fibres, gelatin fibres, silk fibroin fibres, one or more fibrin fibres, chitosan fibres, starch fibres, alginate fibres, hyaluronan fibres, poloaxmer fibres or a combination thereof. The glycosaminoglycan is preferably chondroitin. The cellulose is preferably carboxymethylcellulose, hydroxypropylmethylcellulose or methylcellulose. The poloaxmer is preferably pluronic acid, optionally Pluronic F-127.

If more than one fibre is present in the composition, the population of fibres may be homogenous. In other words, all of the fibres in the population may be the same type of fibre, e.g. cellulose fibres. Alternatively, the population of fibres may be heterogeneous. In other words, the population of fibres may contain different types of fibre, such cellulose fibres and collagen fibres.

The one or more fibres may be any length. The one or more fibres are preferably approximately the same length as the depth of the damage in the tissue which is to be treated using the composition. The length of one or more fibres is preferably designed such that the composition can penetrate a damaged tissue to a prescribed depth. The one or more fibres may be any length. The lower limit of the length of the one or more fibres is typically determined by the diameter of the one or more therapeutic cells. Suitable lengths include, but are not limited to, at least 1µm in length, at least 10µm in length, at least 100µm in length, at least 500µm in length, at least 1mm in length, at least 10mm (1cm) in length, at least 100mm (10cm) in length, at least 500mm (50cm) in length or at least 1000mm (100cm or 1m) in length. The one or more fibres may be even longer. For instance, the one or more fibres may be up to 5m or 10m in length, for instance if being used to repair damage along the human intestinal tract, or even longer if being used in larger animals, such as horses. The length of the one or more fibres is typically determined by their intended use and/or their ability to be manipulated, for instance by a surgeon, by a robot or via some other means, such as magnetically.

The one or more fibres may be charged. The one or more fibres are preferably positively-charged. The one or more fibres are preferably negatively-charged.

The one or more fibres may be magnetic. The one or more fibres may be modified to include one or more magnetic atoms or groups. This allows magnetic targeting of the composition. The magnetic atoms or groups may be paramagnetic or superparamagnetic. Suitable atoms or groups include, but are not limited to, gold atoms, iron atoms, cobalt atoms, nickel atoms and a metal chelating groups, such as nitrilotriacetic acid, containing any of these atoms. The metal chelating group may, for instance, comprise a group selected from -C(=O)O-, -C-O-C-, -C(=O), -NH-, -C(=O)-NH, -C(=O)-CH₂-I, -S(=O)₂- and -S-.

### Biocompatible components

The composition also comprises one or more biocompatible components which comprise a biocompatible adhesive which comprises platelet lysate and attaches the one or more therapeutic cells to the one or more fibres and/or a biocompatible gel which comprises platelet lysate and embeds the one or more therapeutic cells and the one or more fibres.

A component is biocompatible if it does not cause any adverse reactions or side effects when contacted with a damaged tissue.

Any number of biocompatible components may be present in the composition. The composition typically comprises only one component or two components. The composition may comprise more than two components, such as at least 3, at least 5, at least 10, at least 20, at least 30, at least 40, at least 50 components or even more components.

The biocompatible adhesive may attach the one or more therapeutic cells (a) on the surface of the one or more fibres, (b) within the one or more fibres or (c) both on the surface of and within the one or more fibres.

The biocompatible adhesive may be natural or synthetic. Suitable biocompatible adhesives are known in the art. Suitable adhesives include, but are not limited to, fibrin, fibrin gel, integrin, integrin gel, cadherin and cadherin gel.

The one or more biocompatible components preferably comprise a biocompatible gel which embeds the one or more therapeutic cells and the one or more fibres. Suitable biocompatible gels are known in the art. The biocompatible gel may be natural or synthetic. Preferred biocompatible gels include, but are not limited to, a cellulose gel, a collagen gel, a gelatin gel, a fibrin gel, a chitosan gel, a starch gel, an alginate gel, a hyaluronan gel, an agarose gel, a poloaxmer gel or a combination thereof.

The cellulose gel may be formed from any of the celluloses discussed above. The cellulose polymer concentration is preferably from about 1.5% (w/w) to about 4.0% (w/w), such as from about 2.0% (w/w) to about 3.0% (w/w). The cellulose polymer preferably has a molecular weight of from about 450,000 to about 4,000,000, such as from about 500,000 to about 3,500,000, from about 500,000 to about 3,000,000 or from about 750,000 to about 2,500,000 or from about 1000,000 to about 2,000,000.

The poloaxmer gel is preferably a pluronic acid gel, optionally a Pluronic F-127 gel.

The adhesive and/or gel preferably has a viscosity in the range of 1000 to 500,000 mPa·s (cps) at room temperature, such as from about 1500 to about 450,000 mPa·s at room temperature, from about 2000 to about 400,000 mPa·s at room temperature, from about 2500 to about 350,000 mPa·s at room temperature, from about 5000 to about 300,000 mPa·s at room temperature, from about 10,000 to about 250,000 mPa•s at room temperature, from about 50,000 to about 200,000 mPa·s at room temperature or from about 50,000 to about 150,000 mPa·s at room temperature.

Viscosity is a measure of the resistance of the adhesive and/or gel to being deformed by either shear stress or tensile stress. Viscosity can be measured using any method known in the art. Suitable methods include, but are not limited to, using a viscometer or a rheometer.

Room temperature is typically from about 18 °C to about 25 °C, such as from about 19 °C to about 24 °C or from about 20 °C to about 23 °C or from about 21 °C to about 22 °C. Room temperature is preferably any of 18 °C, 19 °C, 20 °C, 21 °C, 22 °C, 23 °C, 24 °C and 25 °C. Viscosity is most preferably measured at 25 °C.

The one or more biocompatible components preferably comprises a biocompatible adhesive which attaches the one or more therapeutic cells to the one or more fibres and a biocompatible gel which embeds the one or more therapeutic cells and the one or more fibres. For instance, the composition may comprise a fibrin gel which attaches the one or more therapeutic cells to the one or more fibres and a cellulose gel which embeds the one or more therapeutic cells and the one or more fibres.

In any of the embodiments discussed above, the biocompatible adhesive and/or the biocompatible gel comprises platelet lysate. For instance, the adhesive and/or the gel may be a platelet lystae gel. Platelet lysate refers to the combination of natural growth factors contained in platelets that has been released through lysing those platelets. Lysis can be accomplished through chemical means (i.e. CaCl₂), osmotic means (use of distilled H₂O) or through freezing/thawing procedures. Platelet lysate can be derived from whole blood as described in U.S. Pat. No. 5,198,357. Platelet lysate is preferably prepared as described in PCT/GB12/052911 (published as WO 2013/076507). For instance, it may be prepared by subjecting a population of platelets to at least one freeze-thaw cycle, wherein the freeze portion of each cycle is carried out at a temperature lower than or equal to - 78 °C.

The adhesive and/or gel preferably comprises (a) platelet lysate, (b) at least one pharmaceutically acceptable polymer and (c) at least one pharmaceutically acceptable positively charged chemical species selected from the group consisting of lysine, arginine, histidine, aspartic acid, glutamic acid, alanine, methionine, proline, serine, asparagine, cysteine, polyamino acids, protamine, aminoguanidine, zinc ions and magnesium ions, wherein the composition is an aqueous gel having a viscosity in the range of 1000 to 500,000 mPa·s (cps) at room temperature. The pharmaceutically acceptable polymer is preferably cellulose or a poloaxmer. It may be any of the celluloses and poloaxmers discussed above.

The platelet lysate is preferably human platelet lysate. Platelet lysate is discussed in more detail below.

### Therapeutic cells

The composition also comprises one or more therapeutic cells. Therapeutic cells are cells which are capable of having a therapeutic effect. Therapeutic cells are typically living cells. Therapeutic cells are typically cells which are capable of repairing damaged tissue. The one or more therapeutic cells are preferably autologous. In other words, the one or more cells are preferably derived from the patient into which the cells will be administered to repair damaged tissue. Alternatively, the one or more cells are preferably allogeneic. In other words, the cells are preferably derived from a patient that is immunologically compatible with the patient into which the cells will be administered to repair damaged tissue. The one or more cells may be semi-allogeneic. Semi-allogeneic populations are typically produced from two or more patients that are immunologically compatible with the patient into which the cells will be administered. In other words, all of the one or more cells are preferably genetically identical with the patient into which they will be administered or sufficiently genetically identical that the cells are immunologically compatible with the patient into which they will be administered.

Any number of cells may be present in the composition. The composition may comprise only one cell. For instance, the composition may comprise one cell and one fibre.

The composition typically comprises more than one cell, such at least 2, at least 5, at least 10, at least 20, at least 30, at least 40, at least 50, at least 100, at least 200, at least 500, at least 1000, at least 2000, at least 5000, at least 10000, at least 50000, at least 100000, at least 5 x 10⁵ , at least 1 x 10⁶, at least 2 x 10⁶, at least 5 x 10⁶, at least 1 x 10⁷, at least 2 x 10⁷, at least 5 x 10⁷, at least 1 x 10⁸ or at least 2 x 10⁸ cells. In some instances, the composition may comprise at least 1.0 x 10⁷, at least 1.0 x 10⁸, at least 1.0 x 10⁹, at least 1.0 x 10¹⁰, at least 1.0 x 10¹¹ or at least 1.0 x 10¹² cells or even more cells.

If more than one cell is present in the composition, the population of cells may be homogenous. In other words, all of the cells in the population may be the same type of cell. Alternatively, the population of cells may be heterogeneous. In other words, the population of cells may contain different types of cells, such MSCs and progenitor cells of mesodermal lineage (PMLs).

The one or more therapeutic cells are preferably one or more progenitor cells of mesodermal lineage (PMLs). PMLs are disclosed in PCT/GB2012/051600 (published as WO 2013/005053). Any of the cells disclosed therein may be used. The PML expresses detectable levels of CD29, CD44, CD73, CD90, CD105 and CD271, but does not express detectable levels of CD14, CD34 and CD45.

The PMLs may advantageously be used to repair damaged tissues in patients. The PMLs are capable of efficiently exerting anti-inflammatory effects in the tissue. The anti-inflammatory effects of the PMLs promote survival, repair and regeneration of the neighbouring cells in the damaged tissue. The cells are also able to exert paracrine effects such as the secretion of angiogenic, chemotactic and anti-apoptotic factors.

As discussed in more detail below, the PMLs are produced from mononuclear cells (MCs), such as peripheral MCs, taken from an individual, such as a human individual. Since the PMLs are produced from MCs, they may be produced easily (such as from peripheral blood) and may be autologous for the patient to be treated and thereby avoid the risk of immunological rejection by the patient.

It is possible, in principle, to produce an unlimited number of PMLs from a single individual, since various samples of MCs (i.e. various samples of blood) may be obtained. It is certainly possible to produce very large numbers of PMLs from a single individual. The PMLs of the invention can therefore be made in large numbers.

The PMLs are produced in clinically relevant conditions, for instance in the absence of trace amounts of endotoxins and other environmental contaminants, as well as animal products such as fetal calf serum. This makes the PMLs particularly suitable for administration to patients.

Since the PMLs are produced from MCs, they are substantially homologous and may be autologous. They also avoid donor-to-donor variation, which frequently occurs with mesenchymal stem cells (MSCs). Numerous populations of PMLs can be produced from a single sample taken from the patient before any other therapy, such as chemotherapy or radiotherapy, has begun. Therefore, the PMLs can avoid any of the detrimental effects of those treatments.

The PMLs can be made quickly. PMLs can be produced from MCs in less than 30 days, such as in about 22 days.

The production of PMLs from MCs avoids the moral and ethical implications involved with using mesenchymal stem cells (MSCs) derived from human embryonic stem cells (hESCs).

The PMLs are typically produced from human MCs. The PMLs are therefore typically human. Alternatively, the PML cells may be produced from MCs from other animals or mammals, for instance from commercially farmed animals, such as horses, cattle, sheep or pigs, from laboratory animals, such as mice or rats, or from pets, such as cats, dogs, rabbits or guinea pigs.

The PMLs can be identified as progenitor cells of mesodermal lineage using standard methods known in the art, including expression of lineage restricted markers, structural and functional characteristics. The PMLs will express detectable levels of cell surface markers known to be characteristic of progenitor cells of mesodermal lineage. In particular, in addition to the markers discussed in more detail below, the PMLs may express α-smooth muscle actin, collagen type I α-chain, GATA6, Mohawk, and vimentin (Sági B et al Stem Cells Dev. 2012 Mar 20; 21(5):814-28).

The PMLs are capable of successfully completing differentiation assays *in vitro* to confirm that they are of mesodermal lineage. Such assays include, but are not limited to, adipogenic differentiation assays, osteogenic differentiation assays and neurogenic differentiation assays (Zaim M et al Ann Hematol. 2012 Aug;91(8):1175-86).

The PMLs are not stem cells. In particular, they are not mesenchymal stem cells (MSCs). They are terminally differentiated. Although they can be forced under the right conditions *in vitro* to differentiating, for instance into cartilage or bone cells, they do not differentiate *in vivo.* The PMLs have their effects by exerting paracrine signalling in the damaged tissue. In particular, the PMLs are preferably capable of inducing anti-flammatory effects in the damaged tissue. This is discussed in more detail below.

The PMLs are typically characterised by a spindle-shaped morphology. The PMLs are typically fibroblast-like, i.e. they have a small cell body with a few cell processes that are long and thin. The cells are typically from about 10 to about 20 µm in diameter.

The PMLs are distinguished from other cells via their marker expression pattern. The PMLs express detectable levels of CD29, CD44, CD73, CD90, CD105 and CD271. The PMLs may overexpress one or more of, such as all of, CD29, CD44, CD73, CD90, CD105 and CD271. The PMLs overexpress one or more of CD29, CD44, CD73, CD90, CD105 and CD271 if they express more than other PMLs and/or MSCs. The PMLs do not express detectable levels of CD14, CD34 and CD45. The PMLs preferably express CD62E (E-selectin) and /or CD62P (P-selectin).

Standard methods known in the art may be used to determine the detectable expression, low expression or lack thereof of the various markers discussed above (and below). Suitable methods include, but are not limited to, immunocytochemistry, immunoassays, flow cytometry, such as fluorescence activated cells sorting (FACS), and polymerase chain reaction (PCR), such as reverse transcription PCR (RT-PCR). Suitable immunoassays include, but are not limited to, Western blotting, enzyme-linked immunoassays (ELISA), enzyme-linked immunosorbent spot assays (ELISPOT assays), enzyme multiplied immunoassay techniques, radioallergosorbent (RAST) tests, radioimmunoassays, radiobinding assays and immunofluorescence. Western blotting, ELISAs and RT-PCR are all quantitative and so can be used to measure the level of expression of the various markers if present. The use of FACS is preferred. Antibodies and fluorescently-labelled antibodies for all of the various markers discussed herein are commercially-available.

The one or more therapeutic cells are preferably one or more immuno-modulatory progenitor (IMP) cells as disclosed in the UK Application being filed concurrently with this application (CTL Ref. IMP1). IMP cells express detectable levels of MIC A/B, CD304 (Neuropilin 1), CD178 (FAS ligand), CD289 (Toll-like receptor 9), CD363 (Sphingosine-1-phosphate receptor 1), CD99, CD181 (C-X-C chemokine receptor type 1; CXCR1), epidermal growth factor receptor (EGF-R), CXCR2 and CD126.

The IMP cells may advantageously be used to repair damaged tissues in patients. The IMP cells are capable of exerting anti-inflammatory effects in the tissue. The anti-inflammatory effects of the IMP cells promote survival, repair and regeneration of the neighbouring cells in the damaged tissue. The cells are also able to exert paracrine effects such as the secretion of angiogenic, chemotactic and anti-apoptotic factors.

The IMP cells are typically produced from human MCs. The IMP cells of the invention are therefore typically human. Alternatively, the IMP cells may be produced from MCs from other animals or mammals, for instance from commercially farmed animals, such as horses, cattle, sheep or pigs, from laboratory animals, such as mice or rats, or from pets, such as cats, dogs, rabbits or guinea pigs.

The IMP cells are also produced from mononuclear cells (MCs), such as peripheral MCs, taken from an individual, such as a human individual. Hence, they have the same advantages as PMLs discussed above. The IMP cells are capable of successfully completing differentiation assays *in vitro* to confirm that they are of mesodermal lineage. Such assays include, but are not limited to, adipogenic differentiation assays, osteogenic differentiation assays and neurogenic differentiation assays (Zaim M et al Ann Hematol. 2012 Aug;91(8):1175-86).

The IMP cells are not stem cells. In particular, they are not MSCs. They are terminally differentiated. Although they can be forced under the right conditions *in vitro* to differentiating, for instance into cartilage or bone cells, they typically do not differentiate *in vivo.* The IMP cells of the invention have their effects by exerting paracrine signalling in the damaged tissue. In particular, the IMP cells are preferably capable of inducing anti-flammatory effects in the damaged tissue. This is discussed in more detail below.

The IMP cells are typically characterised by a spindle-shaped morphology. The IMP cells are typically fibroblast-like, i.e. they have a small cell body with a few cell processes that are long and thin. The cells are typically from about 10 to about 20 µm in diameter.

The IMP cells of the invention are distinguished from known cells, including MSCs, via their marker expression pattern. The IMP cells express detectable levels of MIC A/B, CD304 (Neuropilin 1), CD178 (FAS ligand), CD289 (Toll-like receptor 9), CD363 (Sphingosine-1-phosphate receptor 1), CD99, CD181 (C-X-C chemokine receptor type 1; CXCR1), epidermal growth factor receptor (EGF-R), CXCR2 and CD126. The IMP cells preferably express an increased amount of these markers compared with MSCs. This can be determined by comparing the expression level/amount of the markers in an IMP of the invention with the expression level/amount in an MSC using the same technique under the same conditions. Suitable MSCs are commercially available. The MSC used for comparison is preferably a human MSC. Human MSCs are commercially available from Mesoblast® Ltd, Osiris Therapeutics® Inc. or Lonza®. The human MSC is preferably obtained from Lonza®. Such cells were used for the comparison in the Example. The MSC may be derived from any of the animals or mammals discussed above.

The IMP cells preferably express an increased amount of one or more of MIC A/B, CD304 (Neuropilin 1), CD178 (FAS ligand), CD289 (Toll-like receptor 9), CD363 (Sphingosine-1-phosphate receptor 1), CD99, CD181 (C-X-C chemokine receptor type 1; CXCR1), epidermal growth factor receptor (EGF-R), CXCR2 and CD126 compared with a MSC. The IMP cells preferably express an increased amount of all of the ten markers compared with a MSC.

Any of the methods disclosed above may be used to determine the detectable expression or increased expression of these markers. The expression or increased expression of any of the markers disclosed herein is preferably done using high-throughput FACS (HT-FACS).

The IMP cells preferably demonstrate an antibody mean fluorescence intensity (MFI) of at least 330, such as at least 350 or at least 400, for MIC A/B, an MFI of at least 210, such as at least 250 or at least 300, for CD304 (Neuropilin 1), an MFI of at least 221, such as at least 250 or at least 300, for CD178 (FAS ligand), an MFI of at least 186, such as at least 200 or at least 250, for CD289 (Toll-like receptor 9), an MFI of at least 181, such as at least 200 or at least 250, for CD363 (Sphingosine-1-phosphate receptor 1), an MFI of at least 184, such as at least 200 or at least 250, for CD99, an MFI of at least 300, such as at least 350 or at least 400, for CD181 (C-X-C chemokine receptor type 1; CXCR1), an MFI of at least 173, such as at least 200 or at least 250, for epidermal growth factor receptor (EGF-R), an MFI of at least 236, such as at least 250 or at least 300, for CXCR2 and an MFI of at least 160, such as at least 200 or at least 250, for CD126. Mean fluorescent intensity (MFI) is a measure of intensity, time average energy flux measured in watts per square metre. It is an SI unit. The MFI for each marker is typically measured using HT-FACS. The MFI for each marker is preferably measured using HT-FACS as described in the Example of the UK Application being filed concurrently with this application (CTL Ref. IMP1).

In addition to the ten markers specified above, the IMP cells typically express detectable levels of one or more of the other markers shown in Table 1 in the Example of the UK Application being filed concurrently with this application (CTL Ref. IMP1). The IMP cells may express detectable levels of any number and combination of those markers.

The IMP cells preferably express detectable levels of one or more of CD267, CD47, CD51/CD61, CD49f, CD49d, CD146, CD340, Notch2, CD49b, CD63, CD58, CD44, CD49c, CD105, CD166, HLA-ABC, CD13, CD29, CD49e, CD73, CD81, CD90, CD98, CD147, CD151 and CD276. The IMP cells more preferably express detectable levels of one or more of CD10, CD111, CD267, CD47, CD273, CD51/CD61, CD49f, CD49d, CD146, CD55, CD340, CD91, Notch2, CD175s, CD82, CD49b, CD95, CD63, CD245, CD58, CD108, B2-microglobulin, CD155, CD298, CD44, CD49c, CD105, CD166, CD230, HLA-ABC, CD13, CD29, CD49e, CD59, CD73, CD81, CD90, CD98, CD147, CD151 and CD276. The IMP cells may express detectable levels of any number and combination of these markers. The IMP cells preferably express detectable levels of all of these markers.

The IMP cells preferably express detectable levels of one or more of CD156b, CD61, CD202b, CD130, CD148, CD288, CD337, SSEA-4, CD349 and CD140b. The IMP cells more preferably express detectable levels of one or more of CD156b, CD61, CD202b, CD130, CD148, CD288, CD337, SSEA-4, CD349, CD140b, CD10, CD111, CD267, CD47, CD273, CD51/CD61, CD49f, CD49d, CD146, CD55, CD340, CD91, Notch2, CD175s, CD82, CD49b, CD95, CD63, CD245, CD58, CD108, B2-microglobulin, CD155, CD298, CD44, CD49c, CD105, CD166, CD230, HLA-ABC, CD13, CD29, CD49e, CD59, CD73, CD81, CD90, CD98, CD147, CD151 and CD276. The IMP cells may express detectable levels of any number and combination of these markers. The IMP cells preferably express detectable levels of all of these markers.

The IMP cells preferably express detectable levels of one or more of CD72, CD133, CD192, CD207, CD144, CD41b, FMC7, CD75, CD3e, CD37, CD158a, CD172b, CD282, CD100, CD94, CD39, CD66b, CD158b, CD40, CD35, CD15, PAC-1, CLIP, CD48, CD278, CD5, CD103, CD209, CD3, CD197, HLA-DM, CD20, CD74, CD87, CD129, CDw329, CD57, CD163, TPBG, CD206, CD243 (BD), CD19, CD8, CD52, CD184, CD107b, CD138, CD7, CD50, HLA-DR, CD158e2, CD64, DCIR, CD45, CLA, CD38, CD45RB, CD34, CD101, CD2, CD41a, CD69, CD136, CD62P, TCR alpha beta, CD16b, CD1a, ITGB7, CD154, CD70, CDw218a, CD137, CD43, CD27, CD62L, CD30, CD36, CD150, CD66, CD212, CD177, CD142, CD167, CD352, CD42a, CD336, CD244, CD23, CD45RO, CD229, CD200, CD22, CDH6, CD28, CD18, CD21, CD335, CD131, CD32, CD157, CD165, CD107a, CD1b, CD332, CD180, CD65 and CD24. The IMP cells may express detectable levels of any number and combination of these markers. The IMP cells preferably express detectable levels of all of these markers.

The one or more IMP cells may be part of any of the populations disclosed in the UK Application being filed concurrently with this application (CTL Ref. IMP1).

The IMP cells are preferably capable of adhering to a specific, damaged tissue in a patient. Adherence and adhesion assay are known in the art (Humphries, Methods Mol Biol. 2009;522:203-10).

The IMP cells are preferably capable of proliferating in a specific, damaged tissue in a patient. Cell proliferation assays are well known in the art. Such assays are commercially available, for instance from Life Technologies®.

The IMP cells are preferably capable of promoting angiogenesis in a specific, damaged tissue in a patient. Angiogenesis assays are known in the art (Auerback et al., Clin Chem. 2003 Jan;49(1):32-40).

The IMP cells are preferably capable of having anti-inflammatory effects in a damaged tissue of a patient. The ability of the IMP cells of the invention to have anti-inflammatory effects may also be measured using standard assays known in the art. Suitable methods include, but are not limited to, enzyme-linked immunosorbent assays (ELISAs) for the secretion of cytokines, enhanced mixed leukocyte reactions and up-regulation of co-stimulatory molecules and maturation markers, measured by flow cytometry. Specific methods that may be used are disclosed in the Example. The cytokines measured are typically interleukins, such as interleukin-8 (IL-8), selectins, adhesion molecules, such as Intercellular Adhesion Molecule-1 (ICAM-1), and chemoattractant proteins, such as monocyte chemotactic protein-1 (MCP-1) and tumour necrosis factor alpha (TNF-alpha). Assays for these cytokines are commercially-available. Anti-inflammatory factors are preferably detected and measured using the Luminex® assay described in the Examples. Such assays are commercially available from Life Technologies®.

The IMP cells preferably secrete detectable levels of one or more of interleukin-6 (IL-6), IL-8, C-X-C motif chemokine 10 (CXCL10; interferon gamma-induced protein 10; IP-10), Chemokine (C-C motif) ligand 2 (CCL2; monocyte chemotactic protein-1; MCP-1) and Chemokine (C-C motif) ligand 5 (CCL5; regulated on activation, normal T cell expressed and secreted; RANTES). The IMP cells may secrete any number and combination of these factors. The IMP cells preferably secrete all of these markers.

The IMP cells preferably secrete an increased amount of one or more of IL-6, IL-8, IP-10, MCP-1 and RANTES compared with a MSC. The IMP cells may secrete an increased amount of any number and combination of these factors. The IMP cells preferably secrete an increased amount of all of these markers.

The IMP cells preferably secrete a decreased amount of interleukin-10 (IL-10) and/or IL-12 compared with a mesenchymal stem cell MSC. IL-10 and IL-12 are pro-inflammatory cytokines.

The IMP cells will express a variety of different other markers over and above those discussed above. Some of these will assist the IMP cells will their ability to have anti-inflammatory effects in a damaged tissue. Any of the IMP cells of the invention may further express detectable levels of one or more of (i) insulin-like growth factor-1 (IGF-1), (ii) IGF-1 receptor; (iii) C-C chemokine receptor type 1 (CCR1), (iv) stromal cell-derived factor-1 (SDF-1), (v) hypoxia-inducible factor-1 alpha (HIF-1 alpha), (vi) Akt1 and (vii) hepatocyte growth factor (HGF) and/or granulocyte colony-stimulating factor (G-CSF).

IGF-1 receptors promote migration capacity towards an IGF-1 gradient. One of the mechanisms by which IGF-1 increases migration is by up-regulating CXCR4 on the surface of the cells, which makes them more sensitive to SDF-1 signaling. This is discussed above.

CCR1 is the receptor for CCL7 (previously known as MCP3) increases homing and engraftment capacity of MSCs (and so would be expected to have the same effect for the IMP cells of the invention) and can increase the capillary density in injured myocardium through paracrine signalling.

HIF-1 alpha activates pathways that increase oxygen delivery and promote adaptive pro-survival responses. Among the many target genes of HIF-1 alpha are erythropoietin (EPO), endothelin and VEGF (with its receptor Flk-1). IMP cells that express or express an increased amount of HIF-1 alpha will have upregulated expression of paracrine stimuli of for example several vasculogenic growth factors that may promote a more therapeutic subtype. As described in more detail below, the IMP cells of the invention can be preconditioned into a more therapeutic subtype by culturing them under hypoxic conditions (less than 20% oxygen), such as for example about 2% or about 0% oxygen.

Akt1 is an intracellular serine/threonine protein kinase that plays a key role in multiple cellular processes such as glucose metabolism, cell proliferation, apoptosis, transcription and cell migration. Overexpression of Akt1 has been shown to prevent rat MSCs from undergoing apoptosis and will have the same effect in the IMP cells of the invention. Protection from apoptosis will enhance the therapeutic effect of the IMP cells.

The overexpression of HGF by MSCs has been shown to prevent post-ischemic heart failure by inhibition of apoptosis via calcineurin-mediated pathway and angiogenesis. HGF and G-CSF exhibit synergistic effects in this regard. MSCs that have a high expression of HGF and its receptor c-met also have an increased migratory capacity into the damaged tissue, achieved through hormonal, paracrine and autocrine signaling. The same will be true for the IMP cells of the invention expressing HGF and/or G-CSF.

The IMP cells may express detectable levels off one or more of (i) to (vii) defined above. The IMP cells of the invention preferably express an increased amount of one or more of (i) to (vii) compared with MSCs. Quantitative assays for cell markers are described above. The detectable expression of these markers and their level of expression may be measured as discussed above.

Any of the IMP cells may express detectable levels of one or more of (i) vascular endothelial growth factor (VEGF), (ii) transforming growth factor beta (TGF-beta), (iii) insulin-like growth factor-1 (IGF-1), (iv) fibroblast growth factor (FGF), (v) tumour necrosis factor alpha (TNF-alpha), (vi) interferon gamma (IFN-gamma) and (vii) interleukin-1 alpha (IL-1 alpha). Conditioned medium from cells overexpressing VEGF has been shown to alleviate heart failure in a hamster model. Hence, the IMP cells of the invention which express or express an increased amount of VEGF will have the same effect of damaged cardiac tissue.

The IMP cells may express detectable levels of one or more of (i) to (vii). The IMP cells may express an increased amount of one or more of (i) to (vii) compared with MSCs. Quantitative assays for cell markers are described above. The detectable expression of these markers and their level of expression may be measured as discussed above.

In both sets of definitions of (i) to (vii) given above, any combination of one or more of (i) to (vii) may be expressed or expressed in an increased amount. For instance, for each definition of (i) to (vii), the IMP cells may express detectable levels of, or express an increased amount of, (i); (ii); (iii); (iv); (v); (vi); (vii); (i) and (ii); (i) and (iii); (i) and (iv); (i) and (v); (i) and (vi); (i) and (vii); (ii) and (iii); (ii) and (iv); (ii) and (v); (ii) and (vi); (ii) and (vii); (iii) and (iv); (iii) and (v); (iii) and (vi); (iii) and (vii); (iv) and (v); (iv) and (vi); (iv) and (vii); (v) and (vi); (v) and (vii); (vi) and (vii); (i), (ii) and (iii); (i), (ii) and (iv); (i), (ii) and (v); (i), (ii) and (vi); (i), (ii) and (vii); (i,), (iii) and (iv); (i), (iii) and (v); (i), (iii) and (vi); (i), (iii) and (vii); (i), (iv) and (v); (i), (iv) and (vi); (i), (iv) and (vii); (i), (v) and (vi); (i), (v) and (vii); (i), (vi) and (vii); (ii), (iii) and (iv); (ii), (iii) and (v); (ii), (iii) and (vi); (ii), (iii) and (vii); (ii), (iv) and (v); (ii), (iv) and (vi); (ii), (iv) and (vii); (ii), (v) and (vi); (ii), (v) and (vii); (ii), (vi) and (vii); (iii), (iv) and (v); (iii), (iv) and (vi); (iii), (iv) and (vii); (iii), (v) and (vi); (iii), (v) and (vii); (iii), (vi) and (vii); (iv), (v) and (vi); (iv), (v) and (vii); (iv), (vi) and (vii); (v), (vi) and (vii); (i), (ii), (iii) and (iv); (i), (ii), (iii) and (v); (i), (ii), (iii) and (vi); (i), (ii), (iii) and (vii); (i), (ii), (iv) and (v); (i), (ii), (iv) and (vi); (i), (ii), (iv) and (vii); (i), (ii), (v) and (vi); (i), (ii), (v) and (vii); (i), (ii), (vi) and (vii); (i), (iii), (iv) and (v); (i), (iii), (iv) and (vi); (i), (iii), (iv) and (vii); (i), (iii), (v) and (vi); (i), (iii), (v) and (vii); (i), (iii), (vi) and (vii); (i), (iv), (v) and (vi); (i), (iv), (v) and (vii); (i), (iv), (vi) and (vii); (i), (v), (vi) and (vii); (ii), (iii), (iv) and (v); (ii), (iii), (iv) and (vi); (ii), (iii), (iv) and (vii); (ii), (iii), (v) and (vi); (ii), (iii), (v) and (vii); (ii), (iii), (vi) and (vii); (ii), (iv), (v) and (vi); (ii), (iv), (v) and (vii); (ii), (iv), (vi) and (vii); (ii), (v), (vi) and (vii); (iii), (iv), (v) and (vi); (iii), (iv), (v) and (vii); (iii), (iv), (vi) and (vii); (iii), (v), (vi) and (vii); (iv), (v), (vi) and (vii); (i), (ii), (iii), (iv) and (v); (i), (ii), (iii), (iv) and (vi); (i), (ii), (iii), (iv) and (vii); (i), (ii), (iii), (v) and (vi); (i), (ii), (iii), (v) and (vii); (i), (ii), (iii), (vi) and (vii); (i), (ii), (iv), (v) and (vi); (i), (ii), (iv), (v) and (vii); (i), (ii), (iv), (vi) and (vii); (i), (ii), (v), (vi) and (vii); (i), (iii), (iv), (v) and (vi); (i), (iii), (iv), (v) and (vii); (i), (iii), (iv), (vi) and vii); (i), (iii), (v), (vi) and (vii); (i), (iv), (v), (vi) and (vii); (ii), (iii), (iv), (v) and (vi); (ii), iii), (iv), (v) and (vii); (ii), (iii), (iv), (vi) and (vii); (ii), (iii), (v), (vi) and (vii); (ii), (iv), (v), (vi) and (vii); (iii), (iv), (v), (vi) and vii); (i), (ii), (iii), (iv), (v) and (vi); (i), (ii), (iii), (iv), (v) and (vii); (i), (ii), (iii), (iv), (vi) and (vii); (i), (ii), (iii), (v), (vi) and (vii); (i), (ii), (iv), (v), (vi) and (vii); (i), (iii), (iv), (v), (vi) and (vii); (ii), (iii), (iv), (v), (vi) and (vii); or (i), (ii), (iii), (iv), (v), (vi) and (vii). The combinations for each definition of (i) to (vii) are independently selectable from this list.

In addition to any of the markers discussed above, the IMP cells preferably also express detectable levels of, LIF and/or platelet-derived growth factor (PDGF) receptors. The IMP cells of the invention preferably express an increased amount of LIF and/or platelet-derived growth factor (PDGF) receptors compared with mesenchymal stem cells. The PDGF receptors are preferably PDGF-A receptors and/or PSDGF-B receptors. MSCs that have high expression of these receptors can migrate effectively into areas in which platelets have been activated, such as wounds and thrombotic vessels. The same will be true of IMP cells expressing or expressing an increased amount of the receptors.

The PMLs and/or the IMP cells are preferably autologous. In other words, the cells are preferably derived from the patient into which the cells will be administered. Alternatively, the PMLs and/or IMP cells are preferably allogeneic. In other words, the cells are preferably derived from a patient that is immunologically compatible with the patient into which the cells will be administered.

PMLs and/or IMP cells may be isolated using a variety of techniques including antibody-based techniques. Cells may be isolated using negative and positive selection techniques based on the binding of monoclonal antibodies to those surface markers which are present on the PMLs and/or IMP cells (see above). Hence, the PMLs and/or IMP cells may be separated using any antibody-based technique, including fluorescent activated cell sorting (FACS) and magnetic bead separation.

As discussed in more detail below, the PMLs and/or IMP cells may be treated *ex vivo.* Thus the cells may be loaded or transfected with a therapeutic or diagnostic agent and then used therapeutically in the methods of the invention.

The one or more PMLs and/or IMP cells may be produced using any known method. The one or more PMLs are preferably produced using the method described in International Application No. PCT/GB2012/051600 (published as WO 2013/005053). The one or more IMP cells are preferably produced using the method disclosed in the UK Application being filed concurrently with this application (CTL Ref. IMP1).

This typically involves culturing mononuclear cells (MCs) under conditions which induce the MCs to differentiate into PMLs or IMP cells and then harvesting and culturing the PMLs or IMP cells which express the markers discussed above.

Mononuclear cells (MCs) and methods of isolating them are known in the art. The MCs may be primary MCs isolated from bone marrow. The MCs are preferably peripheral blood MCs (PBMCs), such as lymphocytes, monocytes and/or macrophages. PBMCs can be isolated from blood using a hydrophilic polysaccharide, such as Ficoll®. For instance, PBMCs may be isolated from blood using Ficoll-Paque® (a commercially-available density medium) as disclosed in the Example.

Before they are cultured, the MCs may be exposed to a mesenchymal stem cell enrichment cocktail. The cocktail preferably comprises antibodies that recognise CD3, CD14, CD19, CD38, CD66b (which are present on unwanted cells) and a component of red blood cells. Such a cocktail cross links unwanted cells with red blood cells forming immunorosettes which may be removed from the wanted MCs. A preferred cocktail is RosetteSep®.

Conditions suitable for inducing MCs to differentiate into mesenchymal cells (tissue mainly derived from the mesoderm) are known in the art. For instance, suitable conditions are disclosed in Capelli, C., et al. (Human platelet lysate allows expansion and clinical grade production of mesenchymal stromal cells from small samples of bone marrow aspirates or marrow filter washouts. Bone Marrow Transplantation, 2007. 40: p. 785-791). These conditions may also be used to induce MCs to differentiate into PMLs in accordance with the invention.

The method preferably comprises culturing MCs with plasma lysate to induce the MCs to differentiate into PMLs or IMP cells. Platelet lysate refers to the combination of natural growth factors contained in platelets that has been released through lysing those platelets. Lysis can be accomplished through chemical means (i.e. CaCl₂), osmotic means (use of distilled H₂O) or through freezing/thawing procedures. Platelet lysate can be derived from whole blood as described in U.S. Pat. No. 5,198,357. Platelet lysate is preferably prepared as described in PCT/GB12/052911 (published as WO 2013/076507). The plasma lysate is preferably human plasma lysate.

For instance, the MCs may be cultured in a medium comprising platelet lysate for sufficient time to induce the MCs to differentiate into PMLs or IMP cells. The sufficient time is typically from about 15 to about 25 days, preferably about 22 days. The medium preferably comprises about 20% or less platelet lysate by volume, such as about 15% or less by volume or about 10% or less by volume. The medium preferably comprises from about 5% to about 20% of platelet lysate by volume, such as from about 10% to about 15% by volume. The medium preferably comprises about 10% of platelet lysate by volume.

Alternatively, the MCs may be exposed to a mesenchymal enrichment cocktail and then cultured in a medium comprising platelet lysate for sufficient time to induce the MCs to differentiate into PMLs or IMP cells. The sufficient time is typically from about 15 to about 25 days, preferably about 22 days.

The medium is preferably Minimum Essential Medium (MEM). MEM is commercially available from various sources including Sigma-Aldrich. The medium preferably further comprises one or more of heparin, L-glutamine and penicillin/streptavidin (P/S). The L-glutamine may be replaced with GlutaMAX® (which is commercially-available from Life Technologies).

The MCs are tyically cultured under conditions which allow the PMLs or IMP cells to adhere. Suitable conditions are discussed in more detail above.

The MCs are preferably cultured under low oxygen conditions. The MCs are preferably cultured at less than about 20% oxygen (O₂), such as less than about 19%, less than about 18%, less than about 17%, less than about 16%, less than about 15%, less than about 14%, less than about 13%, less than about 12%, less than about 11%, less than about 10%, less than about 9%, less than about 8%, less than about 7%, less than about 6%, less than about 5%, less than about 4%, less than about 3%, less than about 2% or less than about 1% oxygen (O₂). The MCs are preferably cultured at from about 0% to about 19% O₂, such as from about 1% to about 15% O₂, from about 2% to about 10% O₂ or from about 5% to about 8% O₂. The MCs are most preferably cultured at about 0% O₂. The figures for % oxygen (or % O₂) quoted above relate to % by volume of oxygen in the gas supplied to the cells during culture, for instance by the cell incubator. It is possible that some oxygen may leak into the incubator or enter when the door is opened.

The MCs are most preferably cultured in the presence of platelet lysate and under low oxygen conditions. This combination mimics the natural conditions in the damaged tissue and so result in healthier and more therapeutically potent cells. Conventional cell culture is performed in 20% or 21% oxygen (approximately the atmospheric content) but there is no place in the human body that has this oxygen level. The epithelial cells in the lungs would "see" this oxygen level, but once the oxygen is dissolved and leaves the lungs, it decreases to around 17%. From there, it decreases even further to about 1-2% in the majority of the tissues, but being as low as 0.1% in avascular tissues such as the cartilage in the joints.

Producing one or more PMLs or IMP cells also comprises harvesting and culturing PMLs or IMP cells which have the necessary marker expression pattern as discussed above. The PMLs or IMP cells having the necessary marker expression pattern may be harvested using any antibody-based technique, including fluorescent activated cell sorting (FACS) and magnetic bead separation. FACS is preferred.

As will be clear from the discussion above, the production of one or more PMLs or IMP cells is carried out in clinically relevant conditions, i.e. in the absence of trace amounts of endotoxins and other environmental contaminants, such as lipopolysaccharides, lipopeptides and peptidoglycans, etc. This makes the PMLs or IMP cells particularly suitable for administration to patients.

The MCs are preferably obtained from a patient or an allogeneic donor.

The one or more therapeutic cells are preferably human. The one or more therapeutic cells may be derived from any of the animals or mammals discussed above with reference to the source of the PML and IMP cells.

The one or more therapeutic cells are typically cultured *in vitro* before being combined with the other components in the composition. Techniques for culturing cells are well known to a person skilled in the art. The cells are may be cultured under standard conditions of 37°C, 5% CO₂ in medium without serum. The cells are preferably cultured under low oxygen conditions as discussed in more detail below. The cells may be cultured in any suitable flask or vessel, including wells of a flat plate such as a standard 6 well plate. Such plates are commercially available from Fisher scientific, VWR suppliers, Nunc, Starstedt or Falcon. The wells typically have a capacity of from about 1ml to about 4ml.

The flask, vessel or wells within which the population is contained or cultured may be modified to facilitate handling of the cells. For instance, the flask, vessel or wells may be modified to facilitate culture of the cells, for instance by including a growth matrix. The flask, vessel or wells may be modified to allow attachment of the cells or to allow immobilization of the cells onto a surface. One or more surfaces may be coated with extracellular matrix proteins such as laminin or collagen or any other capture molecules that bind to the cells and immobilize or capture them on the surface(s).

The cells may be modified *ex vivo* using any of the techniques described herein. For instance, the population may be transfected or loaded with therapeutic or diagnostics agents. The population may then be used in the methods of treatment discussed in more detail below.

### Preferred compositions

Preferred hybrid compositions of the invention are summarised in the Table below.

| | **Fibre(s)** | **Cell(s)** | **Component(s)** | **Format** |
|---|---|---|---|---|
| 1 | PLGA | MSC, MPC, IMP, PML or mixture thereof | Fibrin (gel) | Cell(s) attached to the fibre(s) |
| 2 | PLGA | MSC, MPC, IMP, PML or mixture thereof | Integrin | Cell(s) attached to the fibre(s) |
| 3 | PLGA | MSC, MPC, IMP, PML or mixture thereof | Cadherin | Cell(s) attached to the fibre(s) |
| 4 | PLGA | MSC, MPC, IMP, PML or mixture thereof | Fibrin (gel) | Cell(s) embedded in the component(s) |
| 5 | PLGA | MSC, MPC, IMP, PML or mixture thereof | Integrin | Cell(s) embedded in the component(s) |
| 6 | PLGA | MSC, MPC, IMP, PML or mixture thereof | Cadherin | Cell(s) embedded in the component(s) |

### Medicaments, methods and therapeutic use

The hybrid composition of the invention may be used in a method of therapy of the human or animal body. Thus the invention provides a hybrid composition of the invention for use in a method of treatment of the human or animal body by therapy. In particular, the invention concerns using the hybrid composition of the invention to repair a damaged tissue in a patient. The invention also concerns using a hybrid composition of the invention to treat a cardiac, bone, cartilage, tendon, ligament, liver, kidney or lung injury or disease in the patient.

The invention provides a method of repairing a damaged tissue in a patient, comprising contacting the damaged tissue with a hybrid composition of the invention, wherein the composition comprises a therapeutically effective number of cells, and thereby treating the damaged tissue in the patient. The invention also provides a hybrid composition of the invention for use in repairing a damaged tissue in the patient. The invention also provides use of a hybrid composition of the invention in the manufacture of a medicament for repairing a damaged tissue in a patient.

The tissue is preferably derived from the mesoderm. The tissue is more preferably cardiac, bone, cartilage, tendon, ligament, liver, kidney or lung tissue.

The damage to the tissue may be caused by injury or disease. The injury or disease is preferably a a cardiac, bone, cartilage, tendon, ligament, liver, kidney or lung injury or disease in a patient. The invention therefore provides a method of treating a cardiac, bone, cartilage, tendon, ligament, liver, kidney or lung injury or disease in a patient, comprising administering to the patient a a hybrid composition of the invention, wherein the composition comprises a therapeutically effective number of cells, and thereby treating the injury or disease in the patient. The invention also provides a hybrid composition of the invention for use in treating a cardiac, bone, cartilage, tendon, ligament, liver, kidney or lung injury or disease in a patient. The invention also provides use of a hybrid composition of the invention in the manufacture of a medicament for treating a cardiac, bone, cartilage, tendon, ligament, liver, kidney or lung injury or disease in a patient.

The cardiac injury or disease is preferably selected from myocardial infarct (MI), left ventricular hypertrophy, right ventricular hypertrophy, emboli, heart failure, congenital heart deficit, heart valve disease, arrhythmia and myocarditis.

MI increases the levels of VEGF and EPO released by the myocardium. Furthermore, MI is associated with an inflammatory reaction and infarcted tissue also releases macrophage migration inhibitory factor (MIF), interleukin (IL-6) and KC/Gro-alpha. CCL7 (previously known as MCP3), CXCL1, CXCL2 are significantly upregulated in the heart following myocardial infarct (MI) and might be implicated in regulating engraftment and homing of MSCs to infarcted myocardium.

In a myocardial infarct mice model, IL-8 was shown to highly up-regulate gene expression primarily in the first 2 days post-MI. Remarkably, the increased IL-8 expression was located predominantly in the infarcted area and the border zone, and only to a far lesser degree in the spared myocardium. By activating CXCR2, MIF displays chemokine-like functions and acts as a major regulator of inflammatory cell recruitment and atherogenesis.

The bone disease or injury is preferably selected from fracture, Salter-Harris fracture, greenstick fracture, bone spur, craniosynostosis, Coffin-Lowry syndrome, fibrodysplasia ossificans progressive, fibrous dysplasia, Fong Disease (or Nail-patella syndrome), hypophosphatasia, Klippel-Feil syndrome, Metabolic Bone Disease, Nail-patella syndrome, osteoarthritis, osteitis deformans (or Paget's disease of bone), osteitis fibrosa cystica (or Osteitis fibrosa or Von Recklinghausen's disease of bone), osteitis pubis, condensing osteitis (or osteitis condensans), osteitis condensans ilii, osteochondritis dissecans, osteogenesis imperfecta, osteomalacia, osteomyelitis, osteopenia, osteopetrosis, osteoporosis, osteonecrosis, porotic hyperostosis, primary hyperparathyroidism, renal osteodystrophy, bone cancer, a bone lesion associated with metastatic cancer, Gorham Stout disease, primary hyperparathyroidism, periodontal disease, and aseptic loosening of joint replacements. The bone cancer can be Ewing sarcoma, multiple myeloma, osteosarcoma (giant tumour of the bone), osteochondroma or osteoclastoma. The metastatic cancer that results in a bone lesion can be breast cancer, prostate cancer, kidney cancer, lung cancer and/or adult T-cell leukemia.

In all instances, the one or more therapeutic cells are preferably derived from the patient or an allogeneic donor. Deriving the cells from the patient should ensure that the cells are themselves not rejected by the patient's immune system. Any difference between the donor and recipient will ultimately cause clearance of the cells, but not before they have repaired at least a part of the damaged tissue.

The invention involves a therapeutically effective number of therapeutic cells. A therapeutically effective number is a number which ameliorates one or more symptoms of the damage, disease or injury. A therapeutically effective number is preferably a number which repairs the damaged tissue or treats the disease or injury. Suitable numbers are discussed in more detail below.

The hybrid composition of the invention may be administered to any suitable patient. The patient is generally a human patient. The patient may be any of the animals or mammals mentioned above with reference to the source of the PML and IMP cells.

The patient may be an infant, a juvenile or an adult. The patient may be known to have a damaged tissue or is suspected of having a damaged tissue. The patient may be susceptible to, or at risk from, the relevant disease or injury. For instance, the patient may be genetically predisposed to heart failure.

The invention may be used in combination with other means of, and substances for, repairing damaged tissue or providing pain relief. In some cases, the hybrid composition of the invention or a liposome or microbubble composition of the invention may be administered simultaneously, sequentially or separately with other substances which are intended for repairing the damaged tissue or for providing pain relief. The hybrid composition of the invention may be used in combination with existing treatments for damaged tissue and may, for example, be simply mixed with such treatments. Thus the invention may be used to increase the efficacy of existing treatments of damaged tissue.

The invention preferably concerns the use of therapeutic cells loaded or transfected with a therapeutic and/or diagnostic agent. A therapeutic agent may help to repair the damaged tissue. A diagnostic agent, such as a fluorescent molecule, may help to identify the location of the composition in the patient. The cells may be loaded or transfected using any method known in the art. The loading of cells may be performed *in vitro* or *ex vivo.* In each case, the cells may simply be in contact with the agent in culture. Alternatively, the cells may be loaded with an agent using delivery vehicle, such as liposomes. Such vehicles are known in the art.

The transfection of cells, such as PMLs or IMP cells, may be performed *in vitro* or *ex vivo.* Alternatively, stable transfection may be perfomed at the MC stage allowing PMLs expressing the transgene to be differentiated from them. The cells are typically transfected with a nucleic acid encoding the agent. For instance, viral particles or other vectors encoding the agent may be employed. Methods for doing this are known in the art.

The nucleic acid gives rise to expression of the agent in the cells. The nucleic acid molecule will preferably comprise a promoter which is operably linked to the sequences encoding the agent and which is active in the PMLs or which can be induced in the cells.

In a particularly preferred embodiment, the nucleic acid encoding the agent may be delivered via a viral particle. The viral particle may comprise a targeting molecule to ensure efficient transfection. The targeting molecule will typically be provided wholly or partly on the surface of the virus in order for the molecule to be able to target the virus to the cells.

Any suitable virus may be used in such embodiments. The virus may, for example, be a retrovirus, a lentivirus, an adenovirus, an adeno-associated virus, a vaccinia virus or a herpes simplex virus. In a particularly preferred embodiment the virus may be a lentivirus. The lentivirus may be a modified HIV virus suitable for use in delivering genes. The lentivirus may be a SIV, FIV, or equine infectious anemia virus (EQIA) based vector. The virus may be a moloney murine leukaemia virus (MMLV). The viruses used in the invention are preferably replication deficient.

Viral particles do not have to be used. Any vector capable of transfecting the cells may be used, such as conventional plasmid DNA or RNA transfection.

Uptake of nucleic acid constructs may be enhanced by several known transfection techniques, for example those including the use of transfection agents. Examples of these agents includes cationic agents, for example, calcium phosphate and DEAE-Dextran and lipofectants, for example, lipofectAmine, fugene and transfectam.

The cells may be loaded or tranfected under suitable conditions. The cells and agent or vector may, for example, be contacted for between five minutes and ten days, preferably from an hour to five days, more preferably from five hours to two days and even more preferably from twelve hours to one day.

In some embodiments, MCs may be recovered from a patient, converted into PMLs and/or IMP cells as discussed above, loaded or transfected *in vitro* and then returned to the same patient. In such instances, the PMLs and/or IMP cells employed in the invention, will be autologous cells and fully matched with the patient. In a preferred case, the cells employed in the invention are recovered from a patient and utilised *ex vivo* and subsequently returned to the same patient.

### Pharmaceutical compositions and administration

The composition of the invention may be formulated using any suitable method. Formulation of cells with standard pharmaceutically acceptable carriers and/or excipients may be carried out using routine methods in the pharmaceutical art. The exact nature of a formulation will depend upon several factors including the cells to be administered and the desired route of administration. Suitable types of formulation are fully described in Remington's Pharmaceutical Sciences, 19th Edition, Mack Publishing Company, Eastern Pennsylvania, USA.

The composition may be administered by any route. Suitable routes include, but are not limited to, intravenous, intramuscular, intraperitoneal or other appropriate administration routes. The composition is preferably administered directly to the damaged tissue.

Compositions may be prepared together with a physiologically acceptable carrier or diluent. The composition may be mixed with an excipient which is pharmaceutically acceptable and compatible with the active ingredient. Suitable excipients are, for example, water, saline, dextrose, glycerol, of the like and combinations thereof.

In addition, if desired, the pharmaceutical compositions of the invention may contain minor amounts of auxiliary substances such as wetting or emulsifying agents, pH buffering agents, and/or adjuvants which enhance effectiveness. The composition preferably comprises human serum albumin.

One suitable carrier or diluents is Plasma-Lyte A®. This is a sterile, nonpyrogenic isotonic solution for intravenous administration. Each 100 mL contains 526 mg of Sodium Chloride, USP (NaCl); 502 mg of Sodium Gluconate (C6H11NaO7); 368 mg of Sodium Acetate Trihydrate, USP (C2H3NaO2•3H2O); 37 mg of Potassium Chloride, USP (KC1); and 30 mg of Magnesium Chloride, USP (MgCl2•6H2O). It contains no antimicrobial agents. The pH is adjusted with sodium hydroxide. The pH is 7.4 (6.5 to 8.0).

The composition is administered in a manner compatible with the dosage formulation and in such amount will be therapeutically effective. The quantity of the therapeutic cells to be administered depends on the subject to be treated, capacity of the subject's immune system and the degree repair desired. Precise amounts of cells required to be administered may depend on the judgement of the practitioner and may be peculiar to each subject.

Any suitable number of cells may be administered to a subject. For example, at least, or about, 0.2 x 10⁶, 0.25 x 10⁶, 0.5 x 10⁶, 1.5 x 10⁶, 4.0 x 10⁶ or 5.0 x 10⁶ cells per kg of patient may administered. For example, at least, or about, 10⁵, 10⁶, 10⁷, 10⁸, 10⁹ cells may be administered. As a guide, the number of cells to be administered may be from 10⁵ to 10⁹, preferably from 10⁶ to 10⁸. Typically, up to 2 x 10⁸ IMP cells are administered to each patient. Any of the specific numbers discussed above may be administered.

The following Example illustrates the invention.

### Example

Hybrid compositions were manufactured according to the schemes shown in Figures 3 to 6.

## Claims

1. A hybrid composition, which comprises:
(a) one or more biocompatible fibres;
(b) one or more therapeutic cells which comprise
one or more progenitor cells of mesodermal lineage (PMLs) which (i) express detectable levels of CD29, CD44, CD73, CD90, CD105 and CD271, (ii) do not express detectable levels of CD14, CD34 and CD45 and (iii) express detectable levels of CD62P (P-selectin) and/or CD62E (E-selectin) or
one or more immuno-modulatory progenitor (IMP) cells expressing detectable levels of MIC A/B, CD304 (Neuropilin 1), CD178 (FAS ligand), CD289 (Toll-like receptor 9), CD363 (Spingosine-1-phosphate receptor 1), CD99, CD181 (C-X-C chemokine receptor type 1; CXCR1), epidermal growth factor receptor (EGF-R), CXCR2 and CD126; and
(c) one or more biocompatible components which comprise a biocompatible adhesive which comprises platelet lysate and attaches the one or more therapeutic cells to the one or more fibres and/or a biocompatible gel which comprises platelet lysate and embeds the one or more therapeutic cells and the one or more fibres.

2. A hybrid composition according to claim 1, wherein the one or more fibres are one or more cellulose fibres, one or more collagen fibres, one or more collagen-glycosaminoglycan fibres, one or more gelatin fibres, one or more silk fibroin fibres, one or more fibrin fibres, one or more chitosan fibres, one or more starch fibres, one or more alginate fibres, one or more hyaluronan fibres, one or more poloaxmer fibres or a combination thereof.

3. A hybrid composition according to claim 2, wherein the glycosaminoglycan is chondroitin.

4. A hybrid composition according to any one of the preceding claims, wherein the biocompatible adhesive attaches the one or more therapeutic cells on the surface of the one or more fibres and/or within the one or more fibres.

5. A hybrid composition according to any one of the preceding claims, wherein the biocompatible adhesive is fibrin gel, integrin or cadherin.

6. A hybrid composition according to any one of the preceding claims, wherein the biocompatible gel is a cellulose gel, a collagen gel, a gelatin gel, a fibrin gel, a chitosan gel, a starch gel, an alginate gel, a hyaluronan gel, an agarose gel, a poloaxmer gel or a combination thereof.

7. A hybrid composition according to any one of the preceding claims the one or more IMP cells express detectable levels of one or more ofCD10, CD111, CD267, CD47, CD273, CD51/CD61, CD49f, CD49d, CD146, CD55, CD340, CD91, Notch2, CD175s, CD82, CD49b, CD95, CD63, CD245, CD58, CD108, B2-microglobulin, CD155, CD298, CD44, CD49c, CD105, CD166, CD230, HLA-ABC, CD13, CD29, CD49e, CD59, CD73, CD81, CD90, CD98, CD147, CD151 and CD276.

8. A hybrid composition according to any one of the preceding claims, wherein the one or more IMP cells express:
(a) detectable levels of one or more of CD156b, CD61, CD202b, CD130, CD148, CD288, CD337, SSEA-4, CD349, CD140b, CD10, CD111, CD267, CD47, CD273, CD51/CD61, CD49f, CD49d, CD146, CD55, CD340, CD91, Notch2, CD175s, CD82, CD49b, CD95, CD63, CD245, CD58, CD108, B2-microglobulin, CD155, CD298, CD44, CD49c, CD105, CD166, CD230, HLA-ABC, CD13, CD29, CD49e, CD59, CD73, CD81, CD90, CD98, CD147, CD151 and CD276; and/or
(b) detectable levels of one or more of CD72, CD133, CD192, CD207, CD144, CD41b, FMC7, CD75, CD3e, CD37, CD158a, CD172b, CD282, CD100, CD94, CD39, CD66b, CD158b, CD40, CD35, CD15, PAC-1, CLIP, CD48, CD278, CD5, CD103, CD209, CD3, CD197, HLA-DM, CD20, CD74, CD87, CD129, CDw329, CD57, CD163, TPBG, CD206, CD243 (BD), CD19, CD8, CD52, CD184, CD107b, CD138, CD7, CD50, HLA-DR, CD158e2, CD64, DCIR, CD45, CLA, CD38, CD45RB, CD34, CD101, CD2, CD41a, CD69, CD136, CD62P, TCR alpha beta, CD16b, CD1a, ITGB7, CD154, CD70, CDw218a, CD137, CD43, CD27, CD62L, CD30, CD36, CD150, CD66, CD212, CD177, CD142, CD167, CD352, CD42a, CD336, CD244, CD23, CD45RO, CD229, CD200, CD22, CDH6, CD28, CD18, CD21, CD335, CD131, CD32, CD157, CD165, CD107a, CD1b, CD332, CD180, CD65 and CD24.

9. A hybrid composition according to any one of the preceding claims, wherein the one or more therapeutic cells are autologous or allogeneic.

10. A method of producing a hybrid composition according to any one of claims 1 to 9, comprising (a) providing one or more biocompatible fibres, one or more therapeutic cells as defined in claim 1 and one or more biocompatible components as defined in claim 1 and (b) attaching the one or more therapeutic cells to the one or more fibres using one or more biocompatible components and/or embedding the one or more therapeutic cells and the one or more fibres in one or more biocompatible components or combining the one or more fibres, the one or more cells and the one or more components and thereby producing a hybrid composition according to any one of claims 1 to 9.

11. A hybrid composition according to any one of claims 1 to 9 for use in a method of repairing a damaged tissue in a patient.

12. A hybrid composition for use according to claim 11,
wherein the method comprises implanting or injecting the hybrid composition into the damaged tissue or adhering or anchoring the hybrid composition to the damaged tissue or
wherein the one or more fibres are approximately the same length as the depth of damage in the tissue.

13. A hybrid composition for use according to claim 11 or 12, wherein the tissue (a) is derived from the mesoderm, (b) is cardiac, bone, cartilage, tendon, ligament, liver, kidney or lung tissue, or (c) the tissue is damaged by injury or disease.

14. A hybrid composition according to any one of claims 1 to 9 for use in a method of treating a cardiac, bone, cartilage, tendon, ligament, liver, kidney or lung injury or disease in a patient.

15. A hybrid composition for use according to claim 14, wherein
(a) the cardiac injury or disease is selected from myocardial infarct, left ventricular hypertrophy, right ventricular hypertrophy, emboli, heart failure, congenital heart deficit, heart valve disease, arrhythmia and myocarditis or
(b) the bone injury or disease is selected from fracture, Salter-Harris fracture, greenstick fracture, bone spur, craniosynostosis, Coffin-Lowry syndrome, fibrodysplasia ossificans progressive, fibrous dysplasia, Fong Disease (or Nail-patella syndrome), hypophosphatasia, Klippel-Feil syndrome, Metabolic Bone Disease, Nail-patella syndrome, osteoarthritis, osteitis deformans (or Paget's disease of bone), osteitis fibrosa cystica (or Osteitis fibrosa or Von Recklinghausen's disease of bone), osteitis pubis, condensing osteitis (or osteitis condensans), osteitis condensans ilii, osteochondritis dissecans, osteogenesis imperfecta, osteomalacia, osteomyelitis, osteopenia, osteopetrosis, osteoporosis, osteonecrosis, porotic hyperostosis, primary hyperparathyroidism, renal osteodystrophy, bone cancer, a bone lesion associated with metastatic cancer, Gorham Stout disease, primary hyperparathyroidism, periodontal disease, and aseptic loosening of joint replacements.

## Patentansprüche

1. Hybridzusammensetzung, umfassend:
(a) eine oder mehrere biokompatible Fasern;
(b) eine oder mehrere therapeutische Zellen, die Folgendes umfassen:
eine oder mehrere Vorläuferzellen der mesodermalen Linie (PMLs), die (i) nachweisbare Mengen von CD29, CD44, CD73, CD90, CD105 und CD271 exprimieren, (ii) keine nachweisbaren Mengen von CD14, CD34 und CD45 exprimieren und (iii) nachweisbare Mengen von CD62P (P-Selektin) und/oder CD62E (E-Selektin) exprimieren oder
eine oder mehrere immunmodulatorische Vorläufer(IMP)-Zellen, die nachweisbare Mengen von MIC A/B, CD304 (Neuropilin 1), CD178 (FAS-Ligand), CD289 (Tollähnlicher Rezeptor 9), CD363 (Spingosin-1-Phosphatrezeptor 1), CD99, CD181 (C-X-C-Chemokinrezeptor Typ 1; CXCR1), epidermalem Wachstumsfaktorrezeptor (EGF-R), CXCR2 und CD 126 exprimieren; und
(c) eine oder mehrere biokompatible Komponenten, die einen biokompatiblen Klebstoff umfassen, der Plättchenlysat umfasst und die eine oder die mehreren therapeutischen Zellen an die eine oder die mehreren Fasern bindet und/oder ein biokompatibles Gel, das Plättchenlysat umfasst und die eine oder die mehreren therapeutischen Zellen und die eine oder die mehreren Fasern einbettet.

2. Hybridzusammensetzung nach Anspruch 1, wobei die eine oder die mehreren Fasern Folgendes sind: eine oder mehrere Cellulosefasern, eine oder mehrere Kollagenfasern, eine oder mehrere Kollagenglykosaminoglykanfasern, eine oder mehrere Gelatinefasern, eine oder mehrere Seidenfibroinfasern, eine oder mehrere Fibrinfasern, eine oder mehrere Chitosanfasern, eine oder mehrere Stärkefasern, eine oder mehrere Alginatfasern, eine oder mehrere Hyaluronanfasern, eine oder mehrere Poloxamerfasern oder eine Kombination davon.

3. Hybridzusammensetzung nach Anspruch 2, wobei das Glykosaminoglykan Chondroitin ist.

4. Hybridzusammensetzung nach einem der vorstehenden Ansprüche, wobei der biokompatible Klebstoff die eine oder die mehreren therapeutischen Zellen auf die Oberfläche der einen oder mehreren Fasern und/oder innerhalb der einen oder mehreren Fasern bindet.

5. Hybridzusammensetzung nach einem der vorstehenden Ansprüche, wobei der biokompatible Klebstoff Fibringel, Integrin oder Cadherin ist.

6. Hybridzusammensetzung nach einem der vorstehenden Ansprüche, wobei das biokompatible Gel Folgendes ist: ein Cellulosegel, ein Kollagengel, ein Gelatinegel, ein Fibringel, ein Chitosangel, ein Stärkegel, ein Alginatgel, ein Hyaluronangel, ein Agarosegel, ein Poloxamergel oder eine Kombination davon.

7. Hybridzusammensetzung nach einem der vorstehenden Ansprüche, die eine oder mehreren IMP-Zellen nachweisbare Mengen von einem oder mehreren der folgenden exprimieren: CD10, CD111, CD267, CD47, CD273, CD51/CD61, CD49f, CD49d, CD146, CD55, CD340, CD91, Notch2, CD175s, CD82, CD49b, CD95, CD63, CD245, CD58, CD108, B2-Mikroglobulin, CD155, CD298, CD44, CD49c, CD105, CD166, CD230, HLA-ABC, CD13, CD29, CD49e, CD59, CD73, CD81, CD90, CD98, CD147, CD151 und CD276.

8. Hybridzusammensetzung nach einem der vorstehenden Ansprüche, wobei die eine oder die mehreren IMP-Zellen Folgendes exprimieren:
(a) nachweisbare Mengen von einem oder mehreren von CD156b, CD61, CD202b, CD130, CD148, CD288, CD337, SSEA-4, CD349, CD140b, CD10, CD111, CD267, CD47, CD273, CD51/CD61, CD49f, CD49d, CD146, CD55, CD340, CD91, Notch2, CD175s, CD82, CD49b, CD95, CD63, CD245, CD58, CD108, B2-Mikroglobulin, CD155, CD298, CD44, CD49c, CD105, CD166, CD230, HLA-ABC, CD13, CD29, CD49e, CD59, CD73, CD81, CD90, CD98, CD147, CD151 und CD276; und/oder
(b) nachweisbare Mengen von einem oder mehreren von CD72, CD133, CD192, CD207, CD144, CD41b, FMC7, CD75, CD3e, CD37, CD158a, CD172b, CD282, CD100, CD94, CD39, CD66b, CD158b, CD40, CD35, CD15, PAC-1, CLIP, CD48, CD278, CD5, CD103, CD209, CD3, CD197, HLA-DM, CD20, CD74, CD87, CD129, CDw329, CD57, CD163, TPBG, CD206, CD243 (BD), CD19, CD8, CD52, CD184, CD107b, CD138, CD7, CD50, HLA-DR, CD158e2, CD64, DCIR, CD45, CLA, CD38, CD45RB, CD34, CD101, CD2, CD41a, CD69, CD136, CD62P, TCR-Alpha-Beta, CD16b, CD1a, ITGB7, CD154, CD70, CDw218a, CD137, CD43, CD27, CD62L, CD30, CD36, CD150, CD66, CD212, CD177, CD142, CD167, CD352, CD42a, CD336, CD244, CD23, CD45RO, CD229, CD200, CD22, CDH6, CD28, CD18, CD21, CD335, CD131, CD32, CD157, CD165, CD107a, CD1b, CD332, CD180, CD65 und CD24.

9. Hybridzusammensetzung nach einem der vorstehenden Ansprüche, wobei die eine oder die mehreren therapeutischen Zellen autolog oder allogen sind.

10. Verfahren zur Herstellung einer Hybridzusammensetzung nach einem der Ansprüche 1 bis 9, umfassend (a) das Bereitstellen einer oder mehrerer biokompatibler Fasern, einer oder mehrerer therapeutischer Zellen, wie definiert in Anspruch 1, und einer oder mehrerer biokompatibler Komponenten, wie definiert in Anspruch 1, und (b) das Binden der einen oder mehreren therapeutischen Zellen an die einen oder mehreren Fasern unter Verwendung einer oder mehrerer biokompatibler Komponenten und/oder das Einbetten der einen oder mehreren therapeutischen Zellen und der einen oder mehreren Fasern in eine oder mehrere biokompatible Komponenten oder das Kombinieren der einen oder mehreren Fasern, der einen oder mehreren Zellen und der einen oder mehreren Komponenten und dadurch Herstellen einer Hybridzusammensetzung nach einem der Ansprüche 1 bis 9.

11. Hybridzusammensetzung nach einem der Ansprüche 1 bis 9 zur Verwendung in einem Verfahren des Reparierens eines beschädigten Gewebes in einem Patienten.

12. Hybridzusammensetzung zur Verwendung nach Anspruch 11,
wobei das Verfahren das Implantieren oder Injizieren der Hybridzusammensetzung in das beschädigte Gewebe oder das Anhaften oder Verankern der Hybridzusammensetzung an/in dem beschädigten Gewebe umfasst, oder
wobei die einen oder mehreren Fasern ungefähr die gleiche Länge wie die Schädigungstiefe im Gewebe aufweisen.

13. Hybridzusammensetzung zur Verwendung nach Anspruch 11 oder 12, wobei das Gewebe (a) vom Mesoderm abgeleitet ist, (b) Herz-, Knochen-, Knorpel-, Sehnen-, Band-, Leber-, Nieren- oder Lungengewebe ist oder (c) das Gewebe durch Verletzung oder Krankheit beschädigt ist.

14. Hybridzusammensetzung nach einem der Ansprüche 1 bis 9 zur Verwendung in einem Verfahren der Behandlung einer Herz-, Knochen-, Knorpel-, Sehnen-, Band-, Leber-, Nieren- oder Lungenschädigung oder -krankheit in einem Patienten.

15. Hybridzusammensetzung zur Verwendung nach Anspruch 14, wobei
(a) die Herzschädigung oder -krankheit ausgewählt ist aus Myokardinfarkt, linksventrikulärer Hypertrophie, rechtsventrikulärer Hypertrophie, Emboli, Herzinsuffizienz, angeborenem Herzfehler, Herzklappenerkrankung, Arrhythmie und Myokarditis oder
(b) die Knochenschädigung oder -krankheit ausgewählt ist aus Fraktur, Salter-Harris-Fraktur, Greenstick-Fraktur, Knochensporn, Kraniosynostose, Coffin-Lowry-Syndrom, Fibrodysplasia Ossificans Progressiva, fibröser Dysplasie, Fong-Krankheit (oder Nagel-Patella-Syndrom), Hypophosphatasie, Klippel-Feil-Syndrom, metabolischer Knochenerkrankung, Nagel-Patella-Syndrom, Osteoarthritis, Osteitis deformans (oder Paget-Krankheit des Knochens), Osteitis fibrosa cystica (oder Osteitis fibrosa oder Von Recklinghausen-Krankheit des Knochens), Osteitis pubis, kondensierender Osteitis (oder Osteitis condensans), Osteitis condensans ilii, Osteochondritis dissecans, Osteogenesis imperfecta, Osteomalazie, Osteomyelitis, Osteopenie, Osteopetrose, Osteoporose, Osteonekrose, porotischer Hyperostose, primärem Hyperparathyreoidismus, renaler Osteodystrophie, Knochenkrebs, einer Knochenläsion in Verbindung mit metastasierendem Krebs, Gorham-Stout-Krankheit, primärem Hyperparathyreoidismus, Parodontalerkrankung und aseptischer Lockerung von Gelenkersätzen.

## Revendications

1. Composition hybride, qui comprend :
(a) au moins une fibre biocompatible ;
(b) au moins une cellule thérapeutique qui comprend
au moins une cellule progénitrice de lignée mésodermique (PML) qui (i) exprime des niveaux détectables de CD29, CD44, CD73, CD90, CD105 et CD271, (ii) n'exprime pas des niveaux détectables de CD14, CD34 et CD45 et (iii) exprime des niveaux détectables de CD62P (P-sélectine) et/ou de CD62E (E-sélectine) ou
au moins une cellule progénitrice immuno-modulatrice (IMP) exprimant des niveaux détectables de MIC A/B, CD304 (Neuropiline 1), CD178 (ligand de FAS), CD289 (récepteur 9 de type Toll), CD363 (récepteur 1 de Spingosine-1-phosphate), CD99, CD181 (récepteur de type 1 de chimiokine C-X-C ; CXCR1), récepteur de facteur de croissance épidermique (EGF-R) ; CXCR2 et CD126 ; et
(c) au moins un composant biocompatible qui comprend un adhésif biocompatible qui comprend un lysat de plaquettes et attache l'au moins une cellule thérapeutique à l'au moins une fibre et/ou un gel biocompatible qui comprend un lysat de plaquettes et incorpore l'au moins une cellule thérapeutique et l'au moins une fibre.

2. Composition hybride selon la revendication 1, dans laquelle l'au moins une fibre constitue au moins une fibre de cellulose, au moins une fibre de collagène, au moins une fibre de glycosaminoglycane- collagène, au moins une fibre de gélatine, au moins une fibre de fibroïne de soie, au moins une fibre de fibrine, au moins une fibre de chitosane, au moins une fibre d'amidon, au moins une fibre d'alginate, au moins une fibre d'hyaluronane, au moins une fibre de poloxamère ou une combinaison de ceux-ci.

3. Composition hybride selon la revendication 2, dans laquelle le glycosaminoglycane est la chondroïtine.

4. Composition hybride selon l'une quelconque des revendications précédentes, dans laquelle l'adhésif biocompatible attache l'au moins une cellule thérapeutique à la surface de l'au moins une fibre et/ou à l'intérieur de l'au moins une fibre.

5. Composition hybride selon l'une quelconque des revendications précédentes, dans laquelle l'adhésif biocompatible est le gel de fibrine, l'intégrine ou la cadhérine.

6. Composition hybride selon l'une quelconque des revendications précédentes, dans laquelle le gel biocompatible est un gel de cellulose, un gel de collagène, un gel de gélatine, un gel de fibrine, un gel de chitosane, un gel d'amidon, un gel d'alginate, un gel d'hyaluronane, un gel d'agarose, un gel de poloxamère ou une combinaison de ceux-ci.

7. Composition hybride selon l'une quelconque des revendications précédentes l'au moins une cellule IMP exprime des niveaux détectables d'au moins un des éléments suivants : CD10, CD111, CD267, CD47, CD273, CD51/CD61, CD49f, CD49d, CD146, CD55, CD340, CD91, Notch2, CD175s, CD82, CD49b, CD95, CD63, CD245, CD58, CD108, B2-microglobuline, CD155, CD298, CD44, CD49c, CD105, CD166, CD230, HLA-ABC, CD13, CD29, CD49e, CD59, CD73, CD81, CD90, CD98, CD147, CD151 et CD276.

8. Composition hybride selon l'une quelconque des revendications précédentes, dans laquelle l'au moins une cellule IMP exprime :
(a) des niveaux détectables d'au moins un des éléments suivants : CD156b, CD61, CD202b, CD130, CD148, CD288, CD337, SSEA-4, CD349, CD140b, CD10, CD111, CD267, CD47, CD273, CD51/CD61, CD49f, CD49d, CD146, CD55, CD340, CD91, Notch2, CD175s, CD82, CD49b, CD95, CD63, CD245, CD58, CD108, B2-microglobuline, CD155, CD298, CD44, CD49c, CD105, CD166, CD230, HLA-ABC, CD13, CD29, CD49e, CD59, CD73, CD81, CD90, CD98, CD147, CD151 et CD276 ; et/ou
(b) des niveaux détectables d'au moins un des éléments suivants : CD72, CD133, CD192, CD207, CD144, CD41b, FMC7, CD75, CD3e, CD37, CD158a, CD172b, CD282, CD100, CD94, CD39, CD66b, CD158b, CD40, CD35, CD15, PAC-1, CLIP, CD48, CD278, CD5, CD103, CD209, CD3, CD197, HLA-DM, CD20, CD74, CD87, CD129, CDw329, CD57, CD163, TPBG, CD206, CD243 (BD), CD19, CD8, CD52, CD184, CD107b, CD138, CD7, CD50, HLA-DR, CD158e2, CD64, DCIR, CD45, CLA, CD38, CD45RB, CD34, CD101, CD2, CD41a, CD69, CD136, CD62P, TCR alpha bêta, CD16b, CD1a, ITGB7, CD154, CD70, CDw218a, CD137, CD43, CD27, CD62L, CD30, CD36, CD150, CD66, CD212, CD177, CD142, CD167, CD352, CD42a, CD336, CD244, CD23, CD45RO, CD229, CD200, CD22, CDH6, CD28, CD18, CD21, CD335, CD131, CD32, CD157, CD165, CD107a, CD1b, CD332, CD180, CD65 et CD24.

9. Composition hybride selon l'une quelconque des revendications précédentes, dans laquelle l'au moins une cellule thérapeutique est autologue ou allogénique.

10. Procédé de production d'une composition hybride selon l'une quelconque des revendications 1 à 9, comprenant (a) la fourniture d'au moins une fibre biocompatible, d'au moins une cellule thérapeutique telle que définie dans la revendication 1 et d'au moins un composant biocompatible tel que défini dans la revendication 1 et (b) la fixation de l'au moins une cellule thérapeutique à l'au moins une fibre à l'aide de l'au moins un composant biocompatible et/ou l'incorporation de l'au moins une cellule thérapeutique et de l'au moins une fibre dans au moins un composant biocompatible ou la combinaison de l'au moins une fibre, de l'au moins une cellule et de l'au moins un composant et ainsi la production d'une composition hybride selon l'une quelconque des revendications 1 à 9.

11. Composition hybride selon l'une quelconque des revendications 1 à 9 destinée à une utilisation dans une méthode de réparation d'un tissu endommagé chez un patient.

12. Composition hybride destinée à une utilisation selon la revendication 11,
dans laquelle la méthode comprend l'implantation ou l'injection de la composition hybride dans le tissu endommagé ou l'adhérence de la composition hybride au tissu endommagé ou de l'ancrage de la composition hybride dans celui-ci ou
dans laquelle l'au moins une fibre est approximativement de la même longueur que la profondeur de l'endommagement dans le tissu.

13. Composition hybride destinée à une utilisation selon la revendication 11 ou 12, dans laquelle le tissu (a) est dérivé du mésoderme, (b) est un tissu cardiaque, osseux, cartilagineux, tendineux, ligamentaire, hépatique, rénal ou pulmonaire ou (c) le tissu est endommagé par une blessure ou une maladie.

14. Composition hybride destinée à une utilisation selon l'une quelconque des revendications 1 à 9 destinée à une utilisation dans une méthode de traitement d'une blessure ou maladie cardiaque, osseuse, cartilagineuse, tendineuse, ligamentaire, hépatique, rénale ou pulmonaire chez un patient.

15. Composition hybride destinée à une utilisation selon la revendication 14, dans laquelle
(a) la blessure ou maladie cardiaque est choisie parmi l'infarctus du myocarde, l'hypertrophie ventriculaire gauche, l'hypertrophie ventriculaire droite, l'embolie, l'insuffisance cardiaque, la déficience cardiaque congénitale, la valvulopathie, l'arythmie et la myocardite ou
(b) la blessure ou maladie osseuse est choisie parmi la fracture, la fracture de Salter-Harris, la fracture de bois vert, ostéophyte, craniosynostose, le syndrome de Coffin-Lowry, la fibrodysplasie ossifiante progressive, la dysplasie fibreuse, la maladie Fong (ou le syndrome onycho patello rénal), l'hypophosphatasie, le syndrome de Klippel-Feil, la maladie osseuse métabolique, le syndrome onycho patello rénal, l'ostéoarthrite, l'ostéite déformante (ou la maladie osseuse de Paget), l'ostéite fibro-kystique (ou Osteitis fibrosa ou la maladie osseuse de Von Recklinghausen), l'ostéite pubienne, l'ostéite condensante (ou osteitis condensans), l'ostéite condensante ilii, l'ostéochondrite disséquante, l'ostéogenèse imparfaite, l'ostéomalacie, l'osteomyélite, l'ostéopénie, l'ostéopétrose, l'ostéoporose, l'ostéonécrose, l'hyperostose porotique, l'hyperparathyroïdie primaire, l'ostéodystrophie rénale, le cancer osseux, une lésion osseuse associée à un cancer métastatique, la maladie de Gorham-Stout, l'hyperparathyroïdie primaire, la maladie péridontale et le descellement aseptique de prothèses articulaires.
